(19) **Europäisches Patentamt / European Patent Office / Office européen des brevets**

(11) **EP 4 138 084 B1**

(12) **EUROPEAN PATENT SPECIFICATION**

(45) Date of publication and mention of the grant of the patent:
**17.04.2024  Bulletin 2024/16**

(21) Application number: **21887860.1**

(22) Date of filing: **27.10.2021**

(51) International Patent Classification (IPC):
*G16C 10/00* (2019.01)   *G16C 20/30* (2019.01)
*G16B 15/30* (2019.01)

(52) Cooperative Patent Classification (CPC):
**G16B 15/30; G16C 20/30**

(86) International application number:
**PCT/CN2021/126540**

(87) International publication number:
**WO 2022/262168 (22.12.2022 Gazette 2022/51)**

(54) **METHOD FOR CHARACTERIZING TASTE PRESENTATION CHARACTERISTICS OF MIXED COMPONENTS, AND USE OF METHOD**

CHARAKTERISIERUNGSVERFAHREN UND ANWENDUNG VON GESCHMACKSEIGENSCHAFTEN VON GEMISCHTEN KOMPONENTEN

PROCÉDÉ DE CARACTÉRISATION DE CARACTÉRISTIQUES DE PRÉSENTATION DE GOÛT DE COMPOSANTS MÉLANGÉS ET UTILISATION DU PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2021  CN 202110662000**

(43) Date of publication of application:
**22.02.2023  Bulletin 2023/08**

(73) Proprietor: **CHINA TOBACCO YUNNAN INDUSTRIAL CO., LTD**
**Wuhua District**
**Kunming**
**Yunnan 650231 (CN)**

(72) Inventors:
• **YANG, Qianxu**
**Kunming**
**Yunnan 650231 (CN)**
• **LEI, Sheng**
**Kunming**
**Yunnan 650231 (CN)**
• **ZHANG, Wei**
**Kunming**
**Yunnan 650231 (CN)**
• **ZHE, Wei**
**Kunming**
**Yunnan 650231 (CN)**

• **ZHU, Baokun**
**Kunming**
**Yunnan 650231 (CN)**
• **TIAN, Ran**
**Kunming**
**Yunnan 650231 (CN)**
• **XIE, Zhiqiang**
**Kunming**
**Yunnan 650231 (CN)**
• **YANG, Hongming**
**Kunming**
**Yunnan 650231 (CN)**
• **ZHOU, Guofu**
**Kunming**
**Yunnan 650231 (CN)**

(74) Representative: **Gille Hrabal**
**Partnerschaftsgesellschaft mbB**
**Patentanwälte**
**Brucknerstraße 20**
**40593 Düsseldorf (DE)**

(56) References cited:
**CN-A- 109 685 134      CN-A- 112 102 886**
**CN-A- 112 102 886      CN-A- 113 380 332**
**US-A1- 2016 271 199**

EP 4 138 084 B1

- HU KE ET AL: "Exploring the Mechanism of Liquid Smoke and Human Taste Perception Based on the Synergy of the Electronic Tongue, Molecular Docking, and Multiple Linear Regression", FOOD BIOPHYSICS, SPRINGER US, BOSTON, vol. 15, no. 4, 13 July 2020 (2020-07-13), pages 482-494, XP037275138, ISSN: 1557-1858, DOI: 10.1007/S11483-020-09632-0 [retrieved on 2020-07-13]
- GOEL ANUKRATI ET AL: "In-silico screening of database for finding potential sweet molecules: A combined data and structure based modeling approach", FOOD CHEMISTRY, ELSEVIER LTD, NL, vol. 343, 4 November 2020 (2020-11-04), XP086428315, ISSN: 0308-8146, DOI: 10.1016/J.FOODCHEM.2020.128538 [retrieved on 2020-11-04]
- RUDRAKSH TUWANI ET AL: "BitterSweet: Building machine learning models for predicting the bitter and sweet taste of small molecules", SCIENTIFIC REPORTS, vol. 9, no. 1, 9 May 2019 (2019-05-09), XP055693215, DOI: 10.1038/s41598-019-43664-y

**Description**

**TECHNICAL FIELD**

The present invention belongs to the technical field of food, and in particular, to a method for characterizing taste characteristics of the mixture of components and an application of using the method for characterizing taste characteristics of cigarette smoke.

**BACKGROUND**

Physiologically speaking, the reason why humans can perceive taste substances such as sour, sweetness and bitterness is that taste micro-molecules specifically bind to the corresponding active regions of taste proteins in the human body, thereby inducing downstream signals to produce corresponding taste sensations. Molecular docking is to study whether two molecules can bind, predict their binding mode, and search for the lowest free energy conformation for stable binding between ligands and receptor active regions on the basis of the three-dimensional structures of two molecules (usually including a micro-molecule ligand and a macro-molecule protein receptor). Based on the previous research, the inventor uses different docking modes of taste molecules in the T1R2-T1R3 protein, and adopts the molecular docking method to construct docking modes of taste micro-molecules including sour, sweetness and bitterness with T1R2-T1R3 protein, extract key parameters of molecular docking results, and the taste discrimination model of sour, sweetness and bitterness taste molecules of a single compound is established according to the key parameters of molecular docking results. See Patent CN112102886A of the applicant. Further publications relating to the perception of taste substances are HU KE ET AL: "Exploring the Mechanism of Liquid Smoke and Human Taste Perception Based on the Synergy of the Electronic Tongue, Molecular Docking, and Multiple Linear Regression", FOOD BIOPHYSICS, DOI: 10.1007/S11483-020-09632-0, GOEL ANUKRA.TI ET AL: "In-silico screening of database for finding potential sweet molecules: A combined data and structure based modeling approach", FOOD CHEMISTRY, DOI: 10.1-016/J.FOODCHEM.2020.128538, and RUDRAKSH TUWANI ET AL: "BitterSweet: Building machine learning models for predicting the bitter and sweet taste of small molecules", SCIENTIFIC REPORTS, DOI: 10.1038/s41598-019-43664-y.

Taste compounds are compounds with a certain taste, such as sweetness, sour, and bitterness. In real life, it is often comprehensive taste perception with compounds mixed together, the comprehensive taste perception is closely related to taste characteristics of its constituents, rather than a simple superposition of taste characteristics of these taste compounds.

There are few studies at present regarding taste characteristics of the mixture of components, some of which use representative compounds to predict sweetness of mixture of components. However, the pleiotropic taste of monomer compounds and complexity of the composition and content of the mixture of components and even their interaction may result in the diversity of taste of the mixture of components, it is difficult to use individual compounds to characterize taste characteristics of the mixture of components. Thus, new parameters must be introduced to characterize and determine taste characteristics of the mixture of components. On the basis of Patent CN112102886A, the present invention introduces new parameters to characterize taste characteristics of the mixture of components, which are used to characterize the overall taste characteristics of cigarette smoke.

**SUMMARY**

The purpose of the present invention is to provide a method for characterizing taste characteristics of the mixture of components and an application of using the method for characterizing taste characteristics of cigarette smoke, through adopting batch processing by computers without the need of subjective intervention, the method is fast and easy to popularize.

The purpose of the present invention is achieved through the following technical solutions.

Unless otherwise specified, the percentages used in the present invention are by weight.

A first aspect of the present invention discloses a computer-implemented method according to claim 1 for characterizing taste characteristics of the mixture of components, including the following steps: use a molecular docking method to dock each monomer in the mixture of components with T1R2-T1R3, and extract docking result parameters; according to the taste discrimination model containing parameters of docking results, calculate the taste probability and value of each monomer component of bitterness, sweetness and sour; then use the comprehensive weighting method to calculate the overall taste probability of bitterness, sweetness and sour of the mixture of components, so as to obtain taste characteristics of the mixture of components.

The specific steps are as follows:

① Molecular docking of monomer components: conduct molecular docking of each monomer component in the mixture of components with T1R2-T1R3, and extract the parameters of docking results;

② Calculate taste probabilities of monomer components: based on the taste discrimination model containing parameters of docking results, calculate the taste probability of bitterness, sweetness and sour of each monomer component;

③ Calculate taste values of monomer components: according to the content of each monomer component in the mixture of components, calculate the value of bitterness, sweetness and sour of each monomer component;

④ Calculate taste characteristics of the mixture of components: use the comprehensive weighting method to calculate the overall taste probability of the mixture of components of bitterness, sweetness and sour, so as to obtain taste characteristics of the mixture of components.

The parameters of the docking results extracted in step① are as follows:

BE is Binding Energy, equaling to the sum of intermolecular energy and torsional free energy; KI is inhibition constant; ImE is Intermolecular Energy, referring to energy among molecules; IE is Internal Energy, referring to energy inside molecules; TE is Torsional Energy; UEE is Unbound System's Energy; RR is Reference RMSD, referring to the mean square deviation of the current structure relative to the reference conformation.

Parameters and explanations of docking results are as follows:

| Name | Abbreviation | Explanation |
| --- | --- | --- |
| Binding Energy | BE | The sum of intermolecular energy and torsional free energy |
| Ki | KI | Inhibition constant |
| Intermolecular Energy | ImE | Energy among molecules |
| Internal Energy | IE | Energy inside molecules |
| Torsional Energy | TE | Torsional Energy |
| Unbound System's Energy | UEE | Unbound System's Energy |
| Reference RMSD | RR | Mean square deviation of the current structure relative to the reference conformation |

The taste probability of bitterness, sweetness and sour of each monomer component in step② are calculated by using the following formula:

$$\hat{P}(k|x) = \frac{P(x|k)P(k)}{P(x)},$$

Among which, $P(x|k)$ is the multivariate normal density equation, the formula is as follows:

$$P(x|k) = \frac{1}{\left((2\pi)^m |\Sigma_k|\right)^{1/2}} exp\left(-\frac{1}{2}(x - \mu_k)\Sigma_k^{-1}(x - \mu_k)^T\right),$$

x is the parameter value of the docking result of a certain monomer component, k is the taste group, including bitterness, sweetness and sour; m is the number of docking result parameters, where m=7;

$\Sigma_k$ is the covariance of the k-th group, $\mu_k$ is the mean of the k-th group, $|\Sigma_k|$ is the determinant value of $\Sigma_k$, $\Sigma_k^{-1}$ is the inverse matrix of $\Sigma_k$; (x - is the transposed matrix; the values of covariance $\Sigma$ and mean $\mu$ of different groups of bitterness, sweetness and sour are as follows:

| Group k | Parameter | | Value | | | | | | |
| --- | --- | --- | --- | --- | --- | --- | --- | --- | --- |
| | | | BE | KI | ImE | IE | TE | UEE | RR |
| Bitterness | Σ | BE | 1.74 | 64.17 | 1.04 | -0.64 | 0.7 | -0.64 | 4.14 |
| | | KI | 64.17 | 48065.02 | 133.22 | 4.53 | -69.17 | 4.53 | 806.36 |
| | | ImE | 1.04 | 133.22 | 2.41 | 0.64 | -1.37 | 0.64 | 7.06 |
| | | IE | -0.64 | 4.53 | 0.64 | 0.97 | -1.28 | 0.97 | 1.11 |
| | | TE | 0.7 | -69.17 | -1.37 | -1.28 | 2.08 | -1.28 | -2.92 |
| | | UEE | -0.64 | 4.53 | 0.64 | 0.97 | -1.28 | 0.97 | 1.11 |
| | | RR | 4.14 | 806.36 | 7.06 | 1.11 | -2.92 | 1.11 | 48.32 |
| | μ | | -6.4 | 113.14 | -7.93 | -1.07 | 1.54 | -1.07 | 91.47 |
| Sweetness | Σ | BE | 1.36 | -105.06 | 1.06 | -1.06 | 0.3 | -1.06 | -1.23 |
| | | KI | -105.06 | 35402.7 | -173.76 | 63.16 | 68.57 | 63.16 | 76.49 |
| | | ImE | 1.06 | -173.76 | 3.29 | -0.04 | -2.22 | -0.04 | 1.35 |
| | | IE | -1.06 | 63.16 | -0.04 | 1.66 | -1.01 | 1.66 | -1.06 |
| | | TE | 0.3 | 68.57 | -2.22 | -1.01 | 2.52 | -1.01 | -2.57 |
| | | UEE | -1.06 | 63.16 | -0.04 | 1.66 | -1.01 | 1.66 | -1.06 |
| | | RR | -1.23 | 76.49 | 1.35 | -1.06 | -2.57 | -1.06 | 231.07 |
| | μ | | -3.04 | 119.05 | -6.43 | -3.42 | 3.39 | -3.42 | 89.44 |
| Sour | Σ | BE | 0.23 | -39.3 | 0.86 | 0.04 | -0.63 | 0.04 | 1.14 |
| | | KI | -39.3 | 36008.6 | -169.87 | -9.21 | 130.57 | -9.21 | -210.71 |
| | | ImE | 0.86 | -169.87 | 4.19 | 0.2 | -3.33 | 0.2 | 5.82 |
| | | IE | 0.04 | -9.21 | 0.2 | 0.01 | -0.16 | 0.01 | 0.31 |
| | | TE | -0.63 | 130.57 | -3.33 | -0.16 | 2.7 | -0.16 | -4.68 |
| | | UEE | 0.04 | -9.21 | 0.2 | 0.01 | -0.16 | 0.01 | 0.31 |
| | | RR | 1.14 | -210.71 | 5.82 | 0.31 | -4.68 | 0.31 | 15.42 |
| | μ | | -3.23 | 47.4 | -5.2 | -0.11 | 1.97 | -0.11 | 93.55 |

P(k) is the prior probability of taste k with the following values: $P(k = bitterness) = 38.46\%$, $P(k = sweetness) = 32.31\%$, $P(k = sour) = 29.23\%$,

$P(x)$ is a normal constant, the formula is as follows: $P(x) = \sum P(x \mid k) P(k)$.

Preferably, calculation of the taste value of bitterness, sweetness and sour of each monomer component in step③ is as follows:

The taste value of a certain monomer in the mixture of components = the content of a monomer in the mixture of components × the taste probability of a monomer in the mixture of components.

Preferably, calculation of taste characteristics of the mixture of components in step④ is as follows:

The overall taste probability of bitterness, sweetness and sour of the mixture of components = sum of taste values of the mixture of components/sum of the content of the mixture of components.

A second aspect of the present disclosure which is not claimed is an application of using the method for characterizing taste characteristics of cigarette smoke.

Beneficial effects of the present invention:

The present invention discloses for the first time the method for characterizing taste characteristics of the mixture of components. The characterization method of the present invention can directly observe docking of each taste micro-molecule in the mixture of components with the sweet protein T1R2-T1R3 by means of external tools, and explore the taste mechanism. The characterization method of the present invention neither does need to obtain each taste micro-molecule substance in the mixture of components, nor to conduct a long period of experiments or artificial sensory evaluation, the calculation is conducted by computers with fast computing and accurate prediction results.

## DETAILED DESCRIPTION OF THE EMBODIMENTS

In order to make the purpose, technical solutions and advantages of the present invention clearer, the present invention will be further illustrated below with reference to specific embodiment. It should be understood that the specific embodiment described herein is only used to explain the present invention, but not to limit the present invention. In case no specific technique or condition is indicated in the embodiment, the technique or condition described in the literature in the art or the product specification is used. The reagents or instruments used without the manufacturer's indication are conventional products that can be obtained through purchase.

The present invention describes a method for characterizing taste characteristics of the mixture of components in cigarette smoke, including the following steps: use a molecular docking method to molecularly dock each monomer in mixture of components of cigarette smoke with T1R2-T1R3, and extract docking result parameters; according to the taste discrimination model containing parameters of docking results, calculate the taste probability and value of each monomer component of bitterness, sweetness and sour; then use the comprehensive weighting method to calculate the overall taste probability of bitterness, sweetness and sour of the mixture of components in cigarette smoke, so as to obtain taste characteristics of the mixture of components in cigarette smoke.

The specific steps are as follows:

① Molecular docking of monomer components: conduct molecular docking of each monomer component in the mixture of components with T1R2-T1R3, and extract the parameters of docking results;

② Calculate taste probabilities of monomer components: based on the taste discrimination model containing parameters of docking results, calculate the taste probability of bitterness, sweetness and sour of each monomer component;

③ Calculate taste values of monomer components: according to the content of each monomer component in the mixture of components, calculate the value of bitterness, sweetness and sour of each monomer component;

④ Calculate taste characteristics of the mixture of components: use the comprehensive weighting method to calculate the overall taste probability of the mixture of components of bitterness, sweetness and sour, so as to obtain taste characteristics of the mixture of components.

The parameters of the docking results extracted in step① are as follows:

BE is Binding Energy, equaling to the sum of intermolecular energy and torsional free energy; KI is inhibition constant; ImE is Intermolecular Energy, referring to energy among molecules; IE is Internal Energy, referring to energy inside molecules; TE is Torsional Energy; UEE is Unbound System's Energy; RR is Reference RMSD, referring to the mean square deviation of the current structure relative to the reference conformation.

Parameters and explanations of docking results are as follows:

| Name | Abbreviation | Explanation |
|---|---|---|
| Binding Energy | BE | The sum of intermolecular energy and torsional free energy |
| Ki | KI | Inhibition constant |
| Intermolecular Energy | ImE | Energy among molecules |
| Internal Energy | IE | Energy inside molecules |
| Torsional Energy | TE | Torsional Energy |
| Unbound System's Energy | UEE | Unbound System's Energy |
| Reference RMSD | RR | Mean square deviation of the current structure relative to the reference conformation |

Calculate the taste probability of bitterness, sweetness and sour of each monomer component in step② by using the following formula:

$$\hat{P}(k|x) = \frac{P(x|k)P(k)}{P(x)},$$

Among which, $P(x|k)$ is the multivariate normal density equation, the formula is as follows:

$$P(x|k) = \frac{1}{\left((2\pi)^m |\Sigma_k|\right)^{1/2}} exp\left(-\frac{1}{2}(x-\mu_k)\Sigma_k^{-1}(x-\mu_k)^T\right),$$

x is the parameter value of the docking result of a certain monomer component, k is the taste group, including bitterness, sweetness and sour; m is the number of docking result parameters, where m=7;

$\Sigma_k$ is the covariance of the k-th group, $\mu_k$ is the mean of the k-th group, $|\Sigma_k|$ is the determinant value of $\Sigma_k$, $\Sigma_k^{-1}$ is the inverse matrix of $\Sigma_k$; $(x - \mu_k)^T$ is the transposed matrix; the values of covariance $\Sigma$ and mean $\mu$ of different groups of bitterness, sweetness and sour are as follows:

| Group k | Parameter | | Value | | | | | | |
|---|---|---|---|---|---|---|---|---|---|
| | | | BE | KI | ImE | IE | TE | UEE | RR |
| Bitterness | $\Sigma$ | BE | 1.74 | 64.17 | 1.04 | -0.64 | 0.7 | -0.64 | 4.14 |
| | | KI | 64.17 | 48065.02 | 133.22 | 4.53 | -69.17 | 4.53 | 806.36 |
| | | ImE | 1.04 | 133.22 | 2.41 | 0.64 | -1.37 | 0.64 | 7.06 |
| | | IE | -0.64 | 4.53 | 0.64 | 0.97 | -1.28 | 0.97 | 1.11 |
| | | TE | 0.7 | -69.17 | -1.37 | -1.28 | 2.08 | -1.28 | -2.92 |
| | | UEE | -0.64 | 4.53 | 0.64 | 0.97 | -1.28 | 0.97 | 1.11 |
| | | RR | 4.14 | 806.36 | 7.06 | 1.11 | -2.92 | 1.11 | 48.32 |
| | $\mu$ | | -6.4 | 113.14 | -7.93 | -1.07 | 1.54 | -1.07 | 91.47 |
| Sweetness | $\Sigma$ | BE | 1.36 | -105.06 | 1.06 | -1.06 | 0.3 | -1.06 | -1.23 |
| | | KI | -105.06 | 35402.7 | -173.76 | 63.16 | 68.57 | 63.16 | 76.49 |
| | | ImE | 1.06 | -173.76 | 3.29 | -0.04 | -2.22 | -0.04 | 1.35 |
| | | IE | -1.06 | 63.16 | -0.04 | 1.66 | -1.01 | 1.66 | -1.06 |
| | | TE | 0.3 | 68.57 | -2.22 | -1.01 | 2.52 | -1.01 | -2.57 |
| | | UEE | -1.06 | 63.16 | -0.04 | 1.66 | -1.01 | 1.66 | -1.06 |
| | | RR | -1.23 | 76.49 | 1.35 | -1.06 | -2.57 | -1.06 | 231.07 |
| | $\mu$ | | -3.04 | 119.05 | -6.43 | -3.42 | 3.39 | -3.42 | 89.44 |
| Sour | $\Sigma$ | BE | 0.23 | -39.3 | 0.86 | 0.04 | -0.63 | 0.04 | 1.14 |
| | | KI | -39.3 | 36008.6 | -169.87 | -9.21 | 130.57 | -9.21 | -210.71 |
| | | ImE | 0.86 | -169.87 | 4.19 | 0.2 | -3.33 | 0.2 | 5.82 |
| | | IE | 0.04 | -9.21 | 0.2 | 0.01 | -0.16 | 0.01 | 0.31 |
| | | TE | -0.63 | 130.57 | -3.33 | -0.16 | 2.7 | -0.16 | -4.68 |
| | | UEE | 0.04 | -9.21 | 0.2 | 0.01 | -0.16 | 0.01 | 0.31 |
| | | RR | 1.14 | -210.71 | 5.82 | 0.31 | -4.68 | 0.31 | 15.42 |
| | $\mu$ | | -3.23 | 47.4 | -5.2 | -0.11 | 1.97 | -0.11 | 93.55 |

$P(k)$ is the prior probability of taste k with the following values: $P(k$ = bitterness) = 38.46%, $P(k$ = sweetness) = 32.31%, $P(k$ = sour) = 29.23%,

P(x) is a normal constant, the formula is as follows: $P(x) = \sum P(x|k)P(k)$.

Calculation of the taste value of bitterness, sweetness and sour of each monomer component in step ③ is as follows: the taste value of a certain monomer in the mixture of components = the content of a monomer in the mixture of components × the taste probability of a monomer in the mixture of components.

Calculation of taste characteristics of the mixture of components in step④ is as follows: the overall probability of bitterness, sweetness and sour of the mixture of components = sum of taste values of the mixture of components/sum of the content of the mixture of components.

**Embodiment**

In the embodiment, cigarette smoke of a certain brand A is used as the mixture of components, and the cigarette smoke is a mixture of 92 main components through analysis (other minor components are ignored). The 92 components and their contents of cigarette smoke are shown in Table 1 and Table 2.

The method for characterizing taste characteristics of the mixture of components of cigarette smoke includes the following steps:

Step 1, Molecular docking of each monomer component in cigarette smoke.

Use AutoDock software, each component in cigarette smoke is docked with the T1R2-T1R3 protein, so as to obtain parameter values of the docking results as shown in Table 1.

Step 2, Determine the taste probability of each component in cigarette smoke.

Substitute the molecular docking result parameter of each component in cigarette smoke into the formula of

$$\hat{P}(k|x) = \frac{P(x|k)P(k)}{P(x)}$$

the taste discrimination model , calculate the taste probability of bitterness, sweetness and sour of each component in cigarette smoke, as shown in Table 2.

Step 3, calculate the taste value of each component in cigarette smoke according to the following formula, and the results are shown in Table 2.

Taste value of a certain monomer in mixture of components of cigarette smoke = the content of the monomer in the mixture of components of cigarette smoke× the taste probability of the monomer in the mixture of components of cigarette smoke.

Step 4, determine taste characteristics of the mixture of components in cigarette smoke, and calculate according to the following formula, the results are shown in Table 3.

The overall taste probability of bitterness, sweetness, and sour of the mixture of components in cigarette smoke = sum of the taste value of the mixture of components in cigarette smoke/sum of the content of the mixture of components in cigarette smoke.

It can be seen from Table 3 that the overall taste of cigarette smoke of a certain brand A is mainly bitter, followed by sour, and sweetness is the lowest, which is consistent with actual sensory evaluation.

Table 1

| No. | Compound | BE | KI | ImE | IE | TE | UEE | RR |
|-----|----------|------|--------|------|-------|------|-------|--------|
| 1 | Phenol | -4.44 | 560.64 | -4.73 | 0.01 | 0.30 | 0.01 | 101.53 |
| 2 | 2-Ethylphenol | -5.05 | 198.90 | -5.65 | -0.06 | 0.60 | -0.06 | 90.52 |
| 3 | 3-Ethylphenol | -4.82 | 293.96 | -5.41 | -0.06 | 0.60 | -0.06 | 90.15 |
| 4 | 4-Ethylphenol | -4.98 | 224.45 | -5.57 | -0.05 | 0.60 | -0.05 | 90.05 |
| 5 | 2,5-Dimethylphenol | -5.11 | 179.53 | -5.41 | 0.04 | 0.30 | 0.04 | 91.18 |
| 6 | 2,6-Dimethylphenol | -5.04 | 202.75 | -5.34 | 0.04 | 0.30 | 0.04 | 90.95 |
| 7 | 2,6-Dimethoxyphenol | -4.69 | 365.11 | -5.58 | -1.00 | 0.89 | -1.00 | 110.56 |
| 8 | Quinol | -4.22 | 805.29 | -4.82 | 0.03 | 0.60 | 0.03 | 89.82 |
| 9 | 2-Methylhydroquinone | -4.47 | 531.36 | -5.06 | -0.04 | 0.60 | -0.04 | 90.80 |
| 10 | Guaiacol | -4.73 | 343.84 | -5.32 | -0.17 | 0.60 | -0.17 | 110.86 |
| 11 | 4-Vinylguaiacol | -5.78 | 57.61 | -6.68 | -0.20 | 0.89 | -0.20 | 90.42 |
| 12 | Eugenol | -5.31 | 128.33 | -6.50 | -0.32 | 1.19 | -0.32 | 111.06 |
| 13 | Isoeugenol | -5.53 | 87.83 | -6.43 | -0.97 | 0.89 | -0.97 | 89.21 |
| 14 | 3-Hydroxybenzaldehyde | -4.94 | 239.65 | -5.54 | -0.01 | 0.60 | -0.01 | 90.11 |
| 15 | Vanillin | -4.90 | 257.49 | -5.79 | -0.71 | 0.89 | -0.71 | 89.21 |

(continued)

| No. | Compound | BE | KI | ImE | IE | TE | UEE | RR |
|-----|----------|-----|-----|-----|-----|-----|-----|-----|
| 16 | Pyridine | -3.75 | 1.77 | -3.75 | 0.00 | 0.00 | 0.00 | 91.01 |
| 17 | 2-Picoline | -4.17 | 874.02 | -4.17 | 0.00 | 0.00 | 0.00 | 100.33 |
| 18 | 3-Picoline | -4.14 | 923.68 | -4.14 | 0.00 | 0.00 | 0.00 | 90.89 |
| 19 | 4-Picoline | -4.22 | 804.54 | -4.22 | 0.00 | 0.00 | 0.00 | 91.04 |
| 20 | 2-Ethylpyridine | -4.66 | 386.49 | -4.95 | -0.06 | 0.30 | -0.06 | 91.20 |
| 21 | 3-Ethylpyridine | -4.46 | 533.93 | -4.76 | -0.06 | 0.30 | -0.06 | 90.94 |
| 22 | 3-Vinylpyridine | -4.50 | 503.76 | -4.80 | -0.06 | 0.30 | -0.06 | 90.91 |
| 23 | 3-Acetoxypyridine | -4.37 | 626.84 | -4.97 | -0.17 | 0.60 | -0.17 | 110.31 |
| 24 | Pyridone | -4.01 | 1.14 | -4.01 | 0.00 | 0.00 | 0.00 | 102.36 |
| 25 | 6-Methyl-3-pyridinol | -4.51 | 491.86 | -4.81 | -0.01 | 0.30 | -0.01 | 92.07 |
| 26 | Pyrazine | -3.52 | 2.65 | -3.52 | 0.00 | 0.00 | 0.00 | 91.81 |
| 27 | 2-Methylpyrazine | -4.26 | 750.35 | -4.26 | 0.00 | 0.00 | 0.00 | 91.78 |
| 28 | Pyrrole | -3.33 | 3.63 | -3.33 | 0.00 | 0.00 | 0.00 | 100.12 |
| 29 | 2-Pyrrolidone | -3.92 | 1.34 | -3.92 | 0.00 | 0.00 | 0.00 | 102.02 |
| 30 | Succinimide | -4.04 | 1.10 | -4.04 | 0.00 | 0.00 | 0.00 | 103.15 |
| 31 | Indole | -4.76 | 324.34 | -4.76 | 0.00 | 0.00 | 0.00 | 90.43 |
| 32 | 3-Methylindole | -5.16 | 165.47 | -5.16 | 0.00 | 0.00 | 0.00 | 90.01 |
| 33 | Nicotine | -5.96 | 42.84 | -6.26 | -0.15 | 0.30 | -0.15 | 90.66 |
| 34 | Myosmine | -5.43 | 105.53 | -5.72 | -0.19 | 0.30 | -0.19 | 89.98 |
| 35 | 2,3'-Bipyridine | -5.83 | 52.95 | -5.83 | 0.00 | 0.00 | 0.00 | 89.15 |
| 36 | Nicotaine | -5.60 | 79.07 | -5.89 | -0.19 | 0.30 | -0.19 | 89.83 |
| 37 | Quinoline | -5.74 | 61.83 | -5.74 | 0.00 | 0.00 | 0.00 | 90.49 |
| 38 | Acetamide | -3.53 | 2.58 | -3.53 | 0.00 | 0.00 | 0.00 | 111.91 |
| 39 | Isovaleramide | -3.83 | 1.56 | -4.43 | -0.07 | 0.60 | -0.07 | 90.27 |
| 40 | Acrylamide | -3.95 | 1.27 | -4.25 | 0.01 | 0.30 | 0.01 | 102.09 |
| 41 | Cyclopentanone | -4.69 | 362.02 | -4.69 | 0.00 | 0.00 | 0.00 | 102.54 |
| 42 | 2-Ethylcyclopentanone | -4.64 | 398.49 | -4.94 | -0.07 | 0.30 | -0.07 | 89.75 |
| 43 | 2-Cyclopentene-1-one | -3.95 | 1.27 | -3.95 | 0.00 | 0.00 | 0.00 | 102.86 |
| 44 | 2-Methyl-2-cyclopentene-1-one | -4.49 | 511.25 | -4.49 | 0.00 | 0.00 | 0.00 | 91.02 |
| 45 | 3-Methyl-2-cyclopentene-1-one | -4.32 | 678.86 | -4.32 | 0.00 | 0.00 | 0.00 | 90.75 |
| 46 | 2,3-Dimethyl-2-cyclopentene-1-one | -4.87 | 269.81 | -4.87 | 0.00 | 0.00 | 0.00 | 90.58 |
| 47 | 3-Ethyl-2-cyclopentene-1-one | -4.41 | 586.51 | -4.71 | -0.05 | 0.30 | -0.05 | 90.25 |
| 48 | 1,2-Cyclopentanedione | -5.00 | 216.24 | -5.00 | 0.00 | 0.00 | 0.00 | 90.76 |
| 49 | 4-Cyclopentene-1,3-dione | -4.18 | 863.15 | -4.18 | 0.00 | 0.00 | 0.00 | 102.48 |
| 50 | Methylcyclopentenolone | -4.43 | 568.55 | -4.73 | 0.03 | 0.30 | 0.03 | 90.58 |
| 51 | Ethylcyclopentenolone | -4.66 | 381.02 | -5.26 | -0.77 | 0.60 | -0.77 | 90.83 |
| 52 | 2-Cyclohexene-1-one | -4.80 | 301.47 | -4.80 | 0.00 | 0.00 | 0.00 | 90.49 |
| 53 | 1-Nonanone | -5.71 | 65.66 | -5.71 | 0.00 | 0.00 | 0.00 | 90.06 |

(continued)

| No. | Compound | BE | KI | ImE | IE | TE | UEE | RR |
|-----|----------|-----|-----|------|------|------|------|------|
| 54 | Solanone | -5.59 | 79.28 | -7.38 | -0.68 | 1.79 | -0.68 | 87.82 |
| 55 | Megastigmatrienone b | -5.98 | 41.34 | -6.88 | -0.35 | 0.89 | -0.35 | 89.15 |
| 56 | Propionic acid | -2.75 | 9.70 | -3.34 | 0.00 | 0.60 | 0.00 | 97.63 |
| 57 | Butyric acid | -2.77 | 9.38 | -3.36 | 0.02 | 0.60 | 0.02 | 97.62 |
| 58 | Valeric acid | -2.95 | 6.88 | -3.85 | -0.07 | 0.89 | -0.07 | 97.17 |
| 59 | 3-Methylvaleric acid | -3.06 | 5.69 | -4.26 | -0.07 | 1.19 | -0.07 | 91.84 |
| 60 | 2-Acrylic | -2.79 | 9.06 | -3.38 | 0.00 | 0.60 | 0.00 | 97.51 |
| 61 | Crotonic acid | -2.98 | 6.51 | -3.58 | -0.03 | 0.60 | -0.03 | 97.42 |
| 62 | 2-Methylfuran | -4.12 | 954.59 | -4.12 | 0.00 | 0.00 | 0.00 | 91.39 |
| 63 | Furfuryl alcohol | -4.00 | 1.17 | -4.60 | -0.78 | 0.60 | -0.78 | 91.63 |
| 64 | Furfural | -4.48 | 517.70 | -4.78 | -0.07 | 0.30 | -0.07 | 102.75 |
| 65 | 3-Furfural | -4.46 | 534.37 | -4.76 | 0.00 | 0.30 | 0.00 | 102.62 |
| 66 | 5-Methylfurfural | -4.78 | 312.02 | -5.08 | -0.07 | 0.30 | -0.07 | 102.66 |
| 67 | Methyl furoate | -4.51 | 496.94 | -5.10 | -0.27 | 0.60 | -0.27 | 91.76 |
| 68 | 2-Acetylfuran | -4.41 | 589.47 | -4.70 | -0.11 | 0.30 | -0.11 | 90.87 |
| 69 | 2(5H)-Furanone | -3.86 | 1.48 | -3.86 | 0.00 | 0.00 | 0.00 | 91.19 |
| 70 | Pineapplefuranone | -4.62 | 412.72 | -4.92 | -0.65 | 0.30 | -0.65 | 110.93 |
| 71 | Maltol | -4.63 | 400.85 | -4.93 | -0.71 | 0.30 | -0.71 | 90.57 |
| 72 | γ-Butyrolactone | -4.33 | 673.98 | -4.33 | 0.00 | 0.00 | 0.00 | 90.62 |
| 73 | α-Angelica lactone | -4.71 | 352.60 | -4.71 | 0.00 | 0.00 | 0.00 | 91.53 |
| 74 | Dihydroactinodiolide | -5.54 | 87.24 | -5.54 | 0.00 | 0.00 | 0.00 | 91.46 |
| 75 | Limonene | -5.13 | 172.32 | -5.43 | -0.15 | 0.30 | -0.15 | 88.82 |
| 76 | Myrcene | -4.51 | 494.98 | -5.70 | -0.24 | 1.19 | -0.24 | 88.42 |
| 77 | β-Ocimene | -4.60 | 423.61 | -5.50 | -0.22 | 0.89 | -0.22 | 88.02 |
| 78 | Alloocimene | -4.88 | 264.18 | -5.48 | -0.23 | 0.60 | -0.23 | 88.14 |
| 79 | Neophytadiene | -5.42 | 105.84 | -9.30 | -0.77 | 3.88 | -0.77 | 87.06 |
| 80 | 1,2-Propanediol | -2.77 | 9.28 | -3.67 | -1.37 | 0.89 | -1.37 | 86.55 |
| 81 | Glycerol | -2.57 | 12.99 | -4.06 | -3.39 | 1.49 | -3.39 | 89.78 |
| 82 | Methyl pyruvate | -2.97 | 6.69 | -3.56 | -0.03 | 0.60 | -0.03 | 85.99 |
| 83 | Glycerol triacetate | -4.38 | 618.92 | -6.76 | -0.74 | 2.39 | -0.74 | 88.17 |
| 84 | Benzyl alcohol | -4.70 | 357.20 | -5.30 | -0.02 | 0.60 | -0.02 | 90.85 |
| 85 | Phenylethanol | -4.37 | 625.69 | -5.27 | -0.09 | 0.89 | -0.09 | 90.55 |
| 86 | Phenylacetonitrile | -4.90 | 255.45 | -5.20 | -0.12 | 0.30 | -0.12 | 89.41 |
| 87 | Styrene | -4.63 | 401.01 | -4.93 | -0.07 | 0.30 | -0.07 | 102.34 |
| 88 | Ethylbenzene | -4.49 | 510.04 | -4.79 | -0.06 | 0.30 | -0.06 | 90.93 |
| 89 | Xylene | -4.53 | 481.67 | -4.53 | 0.00 | 0.00 | 0.00 | 90.41 |
| 90 | 4-Methylbenzaldehyde | -4.88 | 264.82 | -5.18 | -0.05 | 0.30 | -0.05 | 89.13 |
| 91 | Indene | -4.88 | 264.39 | -4.88 | 0.00 | 0.00 | 0.00 | 90.69 |

(continued)

| No. | Compound | BE | KI | ImE | IE | TE | UEE | RR |
|-----|----------|-----|-------|-------|------|------|------|-------|
| 92 | Naphthalene | -5.44 | 103.72 | -5.44 | 0.00 | 0.00 | 0.00 | 89.47 |

Table 2

| No. | Compound | Relative content | Taste probability | | | Taste value | | |
|---|---|---|---|---|---|---|---|---|
| | | | Bitterness | Sweetness | Sour | Bitterness | Sweetness | Sour |
| 1 | Phenol | 1.43454 | 0.5235 | 0.0286 | 0.4480 | 0.7509 | 0.0410 | 0.6427 |
| 2 | 2-Ethylphenol | 0.10541 | 0.8037 | 0.0067 | 0.1896 | 0.0847 | 0.0007 | 0.0200 |
| 3 | 3-Ethylphenol | 0.00002 | 0.8842 | 0.0157 | 0.1001 | 0.0000 | 0.0000 | 0.0000 |
| 4 | 4-Ethylphenol | 0.00003 | 0.9160 | 0.0106 | 0.0734 | 0.0000 | 0.0000 | 0.0000 |
| 5 | 2,5-Dimethylphenol | 0.08892 | 0.8254 | 0.0045 | 0.1701 | 0.0734 | 0.0004 | 0.0151 |
| 6 | 2,6-Dimethylphenol | 0.03163 | 0.8029 | 0.0054 | 0.1917 | 0.0254 | 0.0002 | 0.0061 |
| 7 | 2,6-Dimethoxyphenol | 0.02835 | 0.7652 | 0.0508 | 0.1840 | 0.0217 | 0.0014 | 0.0052 |
| 8 | Quinol | 0.55554 | 0.5416 | 0.1171 | 0.3413 | 0.3009 | 0.0651 | 0.1896 |
| 9 | 2-Methylhydroquinone | 0.06914 | 0.7857 | 0.0614 | 0.1529 | 0.0543 | 0.0042 | 0.0106 |
| 10 | Guaiacol | 0.15695 | 0.2798 | 0.0044 | 0.7159 | 0.0439 | 0.0007 | 0.1124 |
| 11 | 4-Vinylguaiacol | 0.05031 | 0.9409 | 0.0013 | 0.0578 | 0.0473 | 0.0001 | 0.0029 |
| 12 | Eugenol | 0.00446 | 0.2431 | 0.0009 | 0.7560 | 0.0011 | 0.0000 | 0.0034 |
| 13 | Isoeugenol | 0.02448 | 0.9897 | 0.0076 | 0.0027 | 0.0242 | 0.0002 | 0.0001 |
| 14 | 3-Hydroxybenzaldehyde | 0.02355 | 0.7269 | 0.0077 | 0.2654 | 0.0171 | 0.0002 | 0.0063 |
| 15 | Vanillin | 0.01934 | 0.9434 | 0.0389 | 0.0177 | 0.0182 | 0.0008 | 0.0003 |
| 16 | Pyridine | 0.0632 | 0.0169 | 0.0018 | 0.9813 | 0.0011 | 0.0001 | 0.0620 |
| 17 | 2-Picoline | 0.03404 | 0.4773 | 0.0900 | 0.4327 | 0.0162 | 0.0031 | 0.0147 |
| 18 | 3-Picoline | 0.07477 | 0.7127 | 0.1618 | 0.1255 | 0.0533 | 0.0121 | 0.0094 |
| 19 | 4-Picoline | 0.00758 | 0.7293 | 0.1090 | 0.1617 | 0.0055 | 0.0008 | 0.0012 |
| 20 | 2-Ethylpyridine | 0.01274 | 0.8889 | 0.0251 | 0.0860 | 0.0113 | 0.0003 | 0.0011 |
| 21 | 3-Ethylpyridine | 0.05247 | 0.7064 | 0.0478 | 0.2458 | 0.0371 | 0.0025 | 0.0129 |
| 22 | 3-Vinylpyridine | 0.04925 | 0.7173 | 0.0411 | 0.2416 | 0.0353 | 0.0020 | 0.0119 |
| 23 | 3-Acetoxypyridine | 0.17998 | 0.0969 | 0.0066 | 0.8965 | 0.0174 | 0.0012 | 0.1614 |
| 24 | Pyridone | 1.0191 | 0.0073 | 0.0003 | 0.9924 | 0.0075 | 0.0003 | 1.0114 |

(continued)

| No. | Compound | Relative content | Taste probability | | | Taste value | | |
|---|---|---|---|---|---|---|---|---|
| | | | Bitterness | Sweetness | Sour | Bitterness | Sweetness | Sour |
| 25 | 6-Methyl-3-pyridinol | 0.05862 | 0.6324 | 0.0305 | 0.3370 | 0.0371 | 0.0018 | 0.0198 |
| 26 | Pyrazine | 0.00303 | 0.0058 | 0.0010 | 0.9932 | 0.0000 | 0.0000 | 0.0030 |
| 27 | 2-Methylpyrazine | 0.01874 | 0.7184 | 0.0863 | 0.1953 | 0.0135 | 0.0016 | 0.0037 |
| 28 | Pyrrole | 0.01967 | 0.0006 | 0.0001 | 0.9992 | 0.0000 | 0.0000 | 0.0197 |
| 29 | 2-Pyrrolidone | 0.2563 | 0.0054 | 0.0002 | 0.9944 | 0.0014 | 0.0001 | 0.2549 |
| 30 | Succinimide | 0.20971 | 0.0073 | 0.0002 | 0.9925 | 0.0015 | 0.0000 | 0.2081 |
| 31 | Indole | 0.08366 | 0.8347 | 0.0147 | 0.1506 | 0.0698 | 0.0012 | 0.0126 |
| 32 | 3-Methylindole | 0.08381 | 0.9339 | 0.0043 | 0.0618 | 0.0783 | 0.0004 | 0.0052 |
| 33 | Nicotine | 1.20738 | 0.9953 | 0.0007 | 0.0040 | 1.2017 | 0.0009 | 0.0048 |
| 34 | Myosmine | 0.0252 | 0.9908 | 0.0033 | 0.0059 | 0.0250 | 0.0001 | 0.0001 |
| 35 | 2,3'-Bipyridine | 0.14361 | 0.9934 | 0.0007 | 0.0059 | 0.1427 | 0.0001 | 0.0008 |
| 36 | Nicotaine | 0.05695 | 0.9947 | 0.0022 | 0.0032 | 0.0566 | 0.0001 | 0.0002 |
| 37 | Quinoline | 0.02214 | 0.9887 | 0.0010 | 0.0103 | 0.0219 | 0.0000 | 0.0002 |
| 38 | Acetamide | 0.01404 | 0.0002 | 0.0000 | 0.9998 | 0.0000 | 0.0000 | 0.0140 |
| 39 | Isovaleramide | 0.02953 | 0.0119 | 0.0012 | 0.9869 | 0.0004 | 0.0000 | 0.0291 |
| 40 | Acrylamide | 0.00761 | 0.0032 | 0.0001 | 0.9967 | 0.0000 | 0.0000 | 0.0076 |
| 41 | Cyclopentanone | 0.01154 | 0.3778 | 0.0057 | 0.6165 | 0.0044 | 0.0001 | 0.0071 |
| 42 | 2-Ethylcyclopentanone | 0.00211 | 0.7861 | 0.0265 | 0.1874 | 0.0017 | 0.0001 | 0.0004 |
| 43 | 2-Cyclopentene-1-one | 0.06385 | 0.0053 | 0.0002 | 0.9945 | 0.0003 | 0.0000 | 0.0635 |
| 44 | 2-Methyl-2-cyclopentene-1-one | 0.05684 | 0.7602 | 0.0345 | 0.2054 | 0.0432 | 0.0020 | 0.0117 |
| 45 | 3-Methyl-2-cyclopentene-1-one | 0.156 | 0.7415 | 0.0857 | 0.1728 | 0.1157 | 0.0134 | 0.0270 |
| 46 | 2,3-Dimethyl-2-cyclopent ene-1-one | 0.19404 | 0.8580 | 0.0101 | 0.1319 | 0.1665 | 0.0020 | 0.0256 |
| 47 | 3-Ethyl-2-cyclopentene-1 -one | 0.05342 | 0.7135 | 0.0621 | 0.2244 | 0.0381 | 0.0033 | 0.0120 |
| 48 | 1,2-Cyclopentanedione | 0.01755 | 0.8903 | 0.0067 | 0.1030 | 0.0156 | 0.0001 | 0.0018 |

| No. | Compound | Relative content | Taste probability | | | Taste value | | |
|---|---|---|---|---|---|---|---|---|
| | | | Bitterness | Sweetness | Sour | Bitterness | Sweetness | Sour |
| 49 | 4-Cyclopentene- 1,3-dione | 0.07647 | 0.4025 | 0.0671 | 0.5305 | 0.0308 | 0.0051 | 0.0406 |
| 50 | Methylcyclopentenolone | 0.50358 | 0.6393 | 0.0442 | 0.3164 | 0.3220 | 0.0223 | 0.1594 |
| 51 | Ethylcyclopentenolone | 0.1245 | 0.8995 | 0.0863 | 0.0142 | 0.1120 | 0.0107 | 0.0018 |
| 52 | 2-Cyclohexene-1-one | 0.00879 | 0.8428 | 0.0127 | 0.1445 | 0.0074 | 0.0001 | 0.0013 |
| 53 | 1-Nonanone | 0.02447 | 0.9884 | 0.0009 | 0.0106 | 0.0242 | 0.0000 | 0.0003 |
| 54 | Solanone | 0.02797 | 0.9811 | 0.0066 | 0.0123 | 0.0274 | 0.0002 | 0.0003 |
| 55 | Megastigmatrienone b | 0.04247 | 0.9880 | 0.0011 | 0.0109 | 0.0420 | 0.0000 | 0.0005 |
| 56 | Propionic acid | 0.06318 | 0.0001 | 0.0001 | 0.9998 | 0.0000 | 0.0000 | 0.0632 |
| 57 | Butyric acid | 0.02345 | 0.0001 | 0.0001 | 0.9998 | 0.0000 | 0.0000 | 0.0234 |
| 58 | Valeric acid | 0.00385 | 0.0000 | 0.0000 | 1.0000 | 0.0000 | 0.0000 | 0.0038 |
| 59 | 3-Methylvaleric acid | 0.04118 | 0.0000 | 0.0000 | 0.9999 | 0.0000 | 0.0000 | 0.0412 |
| 60 | 2-Acrylic | 0.06393 | 0.0001 | 0.0001 | 0.9998 | 0.0000 | 0.0000 | 0.0639 |
| 61 | Crotonic acid | 0.02175 | 0.0001 | 0.0001 | 0.9999 | 0.0000 | 0.0000 | 0.0217 |
| 62 | 2-Methylfuran | 0.00113 | 0.6590 | 0.2299 | 0.1111 | 0.0007 | 0.0003 | 0.0001 |
| 63 | Furfuryl alcohol | 0.07182 | 0.3694 | 0.0729 | 0.5577 | 0.0265 | 0.0052 | 0.0401 |
| 64 | Furfural | 0.13029 | 0.2865 | 0.0121 | 0.7015 | 0.0373 | 0.0016 | 0.0914 |
| 65 | 3-Furfural | 0.01266 | 0.2244 | 0.0093 | 0.7663 | 0.0028 | 0.0001 | 0.0097 |
| 66 | 5-Methylfurfural | 0.35208 | 0.3578 | 0.0045 | 0.6377 | 0.1260 | 0.0016 | 0.2245 |
| 67 | Methyl furoate | 0.04513 | 0.8592 | 0.0786 | 0.0622 | 0.0388 | 0.0035 | 0.0028 |
| 68 | 2-Acetylfuran | 0.01918 | 0.8433 | 0.0924 | 0.0643 | 0.0162 | 0.0018 | 0.0012 |
| 69 | 2(5H)-Furanone | 0.01717 | 0.0257 | 0.0021 | 0.9723 | 0.0004 | 0.0000 | 0.0167 |
| 70 | Pineapplefuranone | 0.36779 | 0.6483 | 0.0290 | 0.3227 | 0.2384 | 0.0107 | 0.1187 |
| 71 | Maltol | 0.24389 | 0.9044 | 0.0850 | 0.0106 | 0.2206 | 0.0207 | 0.0026 |
| 72 | $\gamma$-Butyrolactone | 0.0064 | 0.7487 | 0.0839 | 0.1675 | 0.0048 | 0.0005 | 0.0011 |

(continued)

| No. | Compound | Relative content | Taste probability | | | Taste value | | |
|---|---|---|---|---|---|---|---|---|
| | | | Bitterness | Sweetness | Sour | Bitterness | Sweetness | Sour |
| 73 | α-Angelica lactone | 0.00887 | 0.7964 | 0.0162 | 0.1873 | 0.0071 | 0.0001 | 0.0017 |
| 74 | Dihydroactinodiolide | 0.00559 | 0.9739 | 0.0016 | 0.0245 | 0.0054 | 0.0000 | 0.0001 |
| 75 | Limonene | 0.13115 | 0.9447 | 0.0070 | 0.0482 | 0.1239 | 0.0009 | 0.0063 |
| 76 | Myrcene | 0.01594 | 0.6244 | 0.0581 | 0.3175 | 0.0100 | 0.0009 | 0.0051 |
| 77 | β-Ocimene | 0.03435 | 0.5030 | 0.0256 | 0.4715 | 0.0173 | 0.0009 | 0.0162 |
| 78 | Alloocimene | 0.04683 | 0.8928 | 0.0177 | 0.0895 | 0.0418 | 0.0008 | 0.0042 |
| 79 | Neophytadiene | 0.03167 | 0.5711 | 0.0110 | 0.4180 | 0.0181 | 0.0003 | 0.0132 |
| 80 | 1,2-Propanediol | 0.05734 | 0.0228 | 0.2568 | 0.7204 | 0.0013 | 0.0147 | 0.0413 |
| 81 | Glycerol | 0.68101 | 0.0019 | 0.9981 | 0.0000 | 0.0013 | 0.6797 | 0.0000 |
| 82 | Methyl pyruvate | 0.00855 | 0.0020 | 0.0021 | 0.9959 | 0.0000 | 0.0000 | 0.0085 |
| 83 | Glycerol triacetate | 0.70826 | 0.5994 | 0.3279 | 0.0727 | 0.4245 | 0.2322 | 0.0515 |
| 84 | Benzyl alcohol | 0.03451 | 0.6018 | 0.0145 | 0.3837 | 0.0208 | 0.0005 | 0.0132 |
| 85 | Phenylethanol | 0.04617 | 0.2485 | 0.0257 | 0.7258 | 0.0115 | 0.0012 | 0.0335 |
| 86 | Phenylacetonitrile | 0.05545 | 0.8858 | 0.0125 | 0.1017 | 0.0491 | 0.0007 | 0.0056 |
| 87 | Styrene | 0.05476 | 0.3211 | 0.0072 | 0.6716 | 0.0176 | 0.0004 | 0.0368 |
| 88 | Ethylbenzene | 0.01065 | 0.7134 | 0.0425 | 0.2441 | 0.0076 | 0.0005 | 0.0026 |
| 89 | Xylene | 0.04513 | 0.7865 | 0.0349 | 0.1786 | 0.0355 | 0.0016 | 0.0081 |
| 90 | 4-Methylbenzaldehyde | 0.1644 | 0.8510 | 0.0116 | 0.1374 | 0.1399 | 0.0019 | 0.0226 |
| 91 | Indene | 0.11001 | 0.8604 | 0.0097 | 0.1298 | 0.0947 | 0.0011 | 0.0143 |
| 92 | Naphthalene | 0.24132 | 0.9736 | 0.0020 | 0.0244 | 0.2349 | 0.0005 | 0.0059 |

EP 4 138 084 B1

Table 3

| Relative content of mixture of components in cigarette smoke | Sum of taste values | | | Taste probability | | |
|---|---|---|---|---|---|---|
| | Bitterness | Sweetness | Sour | Bitterness | Sweetness | Sour |
| 11.6662 | 6.2239 | 1.1881 | 4.2543 | 0.5335 | 0.1018 | 0.3647 |

The above preferred embodiment is only used to illustrate technical solutions of the present invention, rather than to limit the present invention. Although the present invention has been described in detail through the above preferred embodiment, those skilled in the art should understand that various changes in form and details can be made without departing from the scope of the present invention.

## Claims

1. A computer-implemented method for characterizing taste characteristics of a mixture of components, wherein it comprises the following steps: use a molecular docking method to dock each monomer in the mixture of components with T1R2-T1R3, and extract docking result parameters; according to a taste discrimination model containing parameters of docking results, calculate the taste probability and value of each monomer component of bitterness, sweetness and sour; then use an comprehensive weighting method to calculate overall taste probability of bitterness, sweetness and sour of the mixture of components, so as to obtain taste characteristics of the mixture of components; wherein the specific steps are as follows:

   ① Molecular docking of monomer components: conduct molecular docking of each monomer component in the mixture of components with T1R2-T1R3, and extract the parameters of docking results;
   ② Calculate taste probabilities of monomer components: based on the taste discrimination model containing parameters of docking results, calculate the taste probability of bitterness, sweetness and sour of each monomer component;
   ③ Calculate taste values of monomer components: according to the content of each monomer component in the mixture of components, calculate the value of bitterness, sweetness and sour of each monomer component;
   ④ Calculate taste characteristics of the mixture of components: use the comprehensive weighting method to calculate the overall taste probability of the mixture of components of bitterness, sweetness and sour, so as to obtain taste characteristics of the mixture of components; wherein the parameters of the docking results extracted in step① are as follows:

   BE is Binding Energy, equaling to the sum of intermolecular energy and torsional free energy;
   KI is inhibition constant;
   ImE is Intermolecular Energy, referring to energy among molecules;
   IE is Internal Energy, referring to energy inside molecules;
   TE is Torsional Energy;
   UEE is Unbound System's Energy;
   RR is Reference RMSD, referring to the mean square deviation of the current structure relative to the reference conformation; wherein, the taste probability of bitterness, sweetness and sour of each monomer component in step ② is calculated by using the following formula:

$$\hat{P}(k|x) = \frac{P(x|k)P(k)}{P(x)},$$

Among which, $P(x|k)$ is the multivariate normal density equation, the formula is as follows:

$$P(x|k) = \frac{1}{\left((2\pi)^m |\Sigma_k|\right)^{1/2}} exp\left(-\frac{1}{2}(x - \mu_k)\Sigma_k^{-1}(x - \mu_k)^T\right)$$

x is parameter value of the docking result of a certain monomer component, k is the taste group, including bitterness, sweetness and sour; m is the number of docking result parameters, where m=7;

$\Sigma_k$ is the covariance of the k-th group, $\mu_k$ is the mean of the k-th group, $|\Sigma_k|$ is the determinant value of $\Sigma_k$, $\Sigma_k^{-1}$ is the inverse matrix of $\Sigma_k$; $(x - \mu_k)^T$ is the transposed matrix; the values of covariance $\Sigma$ and mean $\mu$ of different groups of bitterness, sweetness and sour are as follows:

| Group k | Parameter | | Value | | | | | | |
|---|---|---|---|---|---|---|---|---|---|
| | | | BE | KI | ImE | IE | TE | UEE | RR |
| Bitterness | $\Sigma$ | BE | 1.74 | 64.17 | 1.04 | -0.64 | 0.7 | -0.64 | 4.14 |
| | | KI | 64.17 | 48065.02 | 133.22 | 4.53 | -69.17 | 4.53 | 806.36 |
| | | ImE | 1.04 | 133.22 | 2.41 | 0.64 | -1.37 | 0.64 | 7.06 |
| | | IE | -0.64 | 4.53 | 0.64 | 0.97 | -1.28 | 0.97 | 1.11 |
| | | TE | 0.7 | -69.17 | -1.37 | -1.28 | 2.08 | -1.28 | -2.92 |
| | | UEE | -0.64 | 4.53 | 0.64 | 0.97 | -1.28 | 0.97 | 1.11 |
| | | RR | 4.14 | 806.36 | 7.06 | 1.11 | -2.92 | 1.11 | 48.32 |
| | $\mu$ | | -6.4 | 113.14 | -7.93 | -1.07 | 1.54 | -1.07 | 91.47 |
| Sweetness | $\Sigma$ | BE | 1.36 | -105.06 | 1.06 | -1.06 | 0.3 | -1.06 | -1.23 |
| | | KI | -105.06 | 35402.7 | -173.76 | 63.16 | 68.57 | 63.16 | 76.49 |
| | | ImE | 1.06 | -173.76 | 3.29 | -0.04 | -2.22 | -0.04 | 1.35 |
| | | IE | -1.06 | 63.16 | -0.04 | 1.66 | -1.01 | 1.66 | -1.06 |
| | | TE | 0.3 | 68.57 | -2.22 | -1.01 | 2.52 | -1.01 | -2.57 |
| | | UEE | -1.06 | 63.16 | -0.04 | 1.66 | -1.01 | 1.66 | -1.06 |
| | | RR | -1.23 | 76.49 | 1.35 | -1.06 | -2.57 | -1.06 | 231.07 |
| | $\mu$ | | -3.04 | 119.05 | -6.43 | -3.42 | 3.39 | -3.42 | 89.44 |
| Sour | | BE | 0.23 | -39.3 | 0.86 | 0.04 | -0.63 | 0.04 | 1.14 |
| | | KI | -39.3 | 36008.6 | -169.87 | -9.21 | 130.57 | -9.21 | -210.71 |
| | | ImE | 0.86 | -169.87 | 4.19 | 0.2 | -3.33 | 0.2 | 5.82 |
| | | IE | 0.04 | -9.21 | 0.2 | 0.01 | -0.16 | 0.01 | 0.31 |
| | | TE | -0.63 | 130.57 | -3.33 | -0.16 | 2.7 | -0.16 | -4.68 |
| | | UEE | 0.04 | -9.21 | 0.2 | 0.01 | -0.16 | 0.01 | 0.31 |
| | | RR | 1.14 | -210.71 | 5.82 | 0.31 | -4.68 | 0.31 | 15.42 |
| | $\mu$ | | -3.23 | 47.4 | -5.2 | -0.11 | 1.97 | -0.11 | 93.55 |

P(k) is the prior probability of taste k with the following values: $P(k = \text{bitterness}) = 38.46\%$, $P(k = \text{sweetness}) = 32.31\%$, $P(k = \text{sour}) = 29.23\%$,

$P(x)$ is a normal constant, the formula is as follows: $P(x) = \sum P(x \mid k) P(k)$.

2. The method according to claim 1, wherein calculation of the taste value of bitterness, sweetness and sour of each monomer component in step③ is as follows:
The taste value of a certain monomer in the mixture of components = the content of a monomer in the mixture of components $\times$ the taste probability of a monomer in the mixture of components.

3. The method according to claim 1, wherein calculation of taste characteristics of the mixture of components in step④ is as follows:
The overall taste probability of bitterness, sweetness and sour of the mixture of components = sum of taste values of the mixture of components/sum of content of the mixture of components.

**Patentansprüche**

1.  Ein computerimplementiertes Verfahren zur Charakterisierung von Geschmackseigenschaften einer Mischung von Komponenten, wobei es die folgenden Schritte umfasst: Verwenden eines molekularen Docking-Verfahrens, um jedes Monomer in der Mischung von Komponenten mit T1R2-T1R3 anzudocken und Docking-Ergebnis-Parameter zu extrahieren; gemäß einem Geschmacksunterscheidungsmodell, das Parameter von Docking-Ergebnissen enthält, Berechnen der Geschmackswahrscheinlichkeit und des Wertes jeder Monomerkomponente von Bitterkeit, Süße und Säure; dann Verwendung eines umfassenden Gewichtungsverfahrens, um die Gesamtgeschmackswahrscheinlichkeit von Bitterkeit, Süße und Säure der Mischung von Komponenten zu berechnen, um so Geschmackseigenschaften der Mischung von Komponenten zu erhalten; wobei die spezifischen Schritte wie folgt sind:

    ① Molekulares Andocken der Monomerkomponente: Durchführen des molekularen Andockens jeder Monomerkomponente in der Mischung der Komponenten mit T1R2-T1R3 und Extrahieren der Parameter von Docking-Ergebnissen;

    02 Berechnen der Geschmackswahrscheinlichkeiten der Monomerkomponenten: auf der Grundlage des Geschmacksunterscheidungsmodells, das Parameter der Docking-Ergebnisse enthält, Berechnen der Geschmackswahrscheinlichkeit von Bitterkeit, Süße und Säure jeder Monomerkomponente;

    ③ Berechnen von Geschmackswerten der Monomerkomponenten: gemäß dem Gehalt jeder Monomerkomponente in der Mischung von Komponenten, Berechnen der Werte für Bitterkeit, Süße und Säure jeder Monomerkomponente;

    (4) Berechnen von Geschmackscharakteristika der Mischung von Komponenten: Verwenden des umfassenden Gewichtungsverfahrens, um die Gesamtgeschmackswahrscheinlichkeit der Mischung von Komponenten von Bitterkeit, Süße und Säure zu berechnen, um so Geschmackseigenschaften der Mischung von Komponenten zu erhalten; wobei Parameter der in Schritt ① extrahierten Docking-Ergebnisse wie folgt sind:

    BE ist Bindungsenergie, die der Summe der intermolekularen Energie und der freien Torsionsenergie entspricht;
    KI ist die Hemmungskonstante;
    ImE ist intermolekulare Energie, die sich auf die Energie zwischen den Molekülen bezieht;
    IE ist interne Energie, die sich auf die Energie innerhalb der Moleküle bezieht;
    TE ist Torsionsenergie;
    UEE ist Energie des ungebundenen Systems;
    RR ist Referenz-RMSD, die sich auf die mittlere quadratische Abweichung der aktuellen Struktur relativ zur Referenzkonformation bezieht; wobei die Geschmackswahrscheinlichkeit von Bitterkeit, Süße und Säure jeder Monomerkomponente in Schritt ② unter Verwendung der folgenden Formel berechnet wird:

$$\hat{P}(k|x) = \frac{P(x|k)P(k)}{P(x)},$$

darunter ist P (x| k) die multivariate normale Dichtegleichung; die Formel lautet wie folgt:

$$P(x|k) = \frac{1}{\left((2\pi)^m |\Sigma_k|\right)^{1/2}} \, exp\left(-\frac{1}{2}(x - \mu_k)\Sigma_k^{-1}(x - \mu_k)^T\right)$$

x ist der Parameterwert des Docking-Ergebnisses einer bestimmten Monomerkomponente, k ist die Geschmacksgruppe, die Bitterkeit, Süße und Säure enthält; m ist die Anzahl der Docking-Ergebnis-Parameter, wobei m=7;

$\Sigma_k$ ist die Kovarianz der k-ten Gruppe, $\mu_k$ ist der Mittelwert der k-ten Gruppe, $|\Sigma_k|$ ist der Determinantenwert von $\Sigma_k$, $\sum_k^{-1}$ ist die inverse Matrix von $\Sigma_k$; $(x - \mu_k)^T$ ist die transponierte Matrix; die Werte der Kovarianz $\Sigma$ und des Mittelwerts $\mu$ der verschiedenen Gruppen von Bitterkeit, Süße und Säure sind wie folgt:

| Gruppe k | Parameter | Wert | | | | | | |
|---|---|---|---|---|---|---|---|---|
| | | | BE | KI | ImE | IE | TE | UEE | RR |
| Bitterkeit | Σ | BE | 1.74 | 64.17 | 1.04 | -0.64 | 0.7 | -0.64 | 4.14 |
| | | KI | 64.17 | 48065.02 | 133.22 | 4.53 | -69.17 | 4.53 | 806.36 |
| | | ImE | 1.04 | 133.22 | 2.41 | 0.64 | -1.37 | 0.64 | 7.06 |
| | | IE | -0.64 | 4.53 | 0.64 | 0.97 | -1.28 | 0.97 | 1.11 |
| | | TE | 0.7 | -69.17 | -1.37 | -1.28 | 2.08 | -1.28 | -2.92 |
| | | UEE | -0.64 | 4.53 | 0.64 | 0.97 | -1.28 | 0.97 | 1.11 |
| | | RR | 4.14 | 806.36 | 7.06 | 1.11 | -2.92 | 1.11 | 48.32 |
| | μ | | -6.4 | 113.14 | -7.93 | -1.07 | 1.54 | -1.07 | 91.47 |
| Süße | Σ | BE | 1.36 | -105.06 | 1.06 | -1.06 | 0.3 | -1.06 | -1.23 |
| | | KI | -105.06 | 35402.7 | -173.76 | 63.16 | 68.57 | 63.16 | 76.49 |
| | | ImE | 1.06 | -173.76 | 3.29 | -0.04 | -2.22 | -0.04 | 1.35 |
| | | IE | -1.06 | 63.16 | -0.04 | 1.66 | -1.01 | 1.66 | -1.06 |
| | | TE | 0.3 | 68.57 | -2.22 | -1.01 | 2.52 | -1.01 | -2.57 |
| | | UEE | -1.06 | 63.16 | -0.04 | 1.66 | -1.01 | 1.66 | -1.06 |
| | | RR | -1.23 | 76.49 | 1.35 | -1.06 | -2.57 | -1.06 | 231.07 |
| | μ | | -3.04 | 119.05 | -6.43 | -3.42 | 3.39 | -3.42 | 89.44 |
| Säure | Σ | BE | 0.23 | -39.3 | 0.86 | 0.04 | -0.63 | 0.04 | 1.14 |
| | | KI | -39.3 | 36008.6 | -169.87 | -9.21 | 130.57 | -9.21 | -210.71 |
| | | ImE | 0.86 | -169.87 | 4.19 | 0.2 | -3.33 | 0.2 | 5.82 |
| | | IE | 0.04 | -9.21 | 0.2 | 0.01 | -0.16 | 0.01 | 0.31 |
| | | TE | -0.63 | 130.57 | -3.33 | -0.16 | 2.7 | -0.16 | -4.68 |
| | | UEE | 0.04 | -9.21 | 0.2 | 0.01 | -0.16 | 0.01 | 0.31 |
| | | RR | 1.14 | -210.71 | 5.82 | 0.31 | -4.68 | 0.31 | 15.42 |
| | μ | | -3.23 | 47.4 | -5.2 | -0.11 | 1.97 | -0.11 | 93.55 |

$P(k)$ ist die vorherige Wahrscheinlichkeit des Geschmacks k mit folgenden Werten: $P(k$ = Bitterkeit) = 38.46%, $P(k$ = Süße) = 32.31%, $P(k$ = Säure) = 29.23%,

$P(x)$ ist eine normale Konstante; die Formel lautet wie folgt: $P(x) = \sum P(x \mid k) P(k)$.

2. Das Verfahren gemäß Anspruch 1, wobei die Berechnung des Geschmackswerts von Bitterkeit, Süße und Säure von jeder Monomerkomponente in Schritt ③ wie folgt ist:
Der Geschmackswert eines bestimmten Monomers in der Mischung von Komponenten = der Gehalt eines Monomers in der Mischung von Komponenten × die Geschmackswahrscheinlichkeit eines Monomers in der Mischung von Komponenten.

3. Das Verfahren gemäß Anspruch 1, wobei die Berechnung der Geschmackseigenschaften der Mischung von Komponenten in Schritt ④ wie folgt ist:
Die Gesamtgeschmackswahrscheinlichkeit von Bitterkeit, Süße und Säure der Mischung von Komponenten = Summe von Geschmackswerten der Mischung von Komponenten /Summe von Inhalt der Mischung von Komponenten.

**Revendications**

1. Méthode mise en oeuvre par ordinateur pour caractériser les caractéristiques gustatives d'un mélange de composants, comprenant les étapes suivantes : utiliser une méthode d'amarrage moléculaire pour amarrer chaque monomère du mélange de composants avec T1R2-T1R3, et extraire les paramètres de résultat d'ancrage ; selon un modèle de discrimination gustative contenant des paramètres de résultat d'amarrage, calculer la probabilité et la valeur gustative d'amertume, de douceur et d'aigreur de chaque composant monomère ; utiliser ensuite une méthode de pondération compréhensive pour calculer la probabilité gustative globale d'amertume, de douceur et d'aigreur du mélange de composants, de manière à obtenir des caractéristiques gustatives du mélange de composants ; dans laquelle les étapes spécifiques sont comme suit :

① Amarrage moléculaire des composants monomères : conduire de l'amarrage moléculaire de chaque composant monomère dans le mélange de composants avec T1R2-T1R3, et extraire les paramètres des résultats d'amarrage ;

② Calculer les probabilités gustatives des composants monomères : sur la base du modèle de discrimination gustative contenant des paramètres des résultats d'amarrage, calculer la probabilité d'amertume, de douceur et d'aigreur de chaque composant monomère ;

③ Calculer les valeurs gustatives des composants monomères : selon le contenu de chaque composant monomère dans le mélange de composants, calculer la valeur d'amertume, de douceur et d'aigreur de chaque composant monomère ;

④ Calculer les caractéristiques gustatives du mélange de composants : utiliser la méthode de pondération compréhensive pour calculer la probabilité gustative globale du mélange de composants d'amertume, de douceur et d'aigreur, de manière à obtenir les caractéristiques gustatives du mélange de composants ; où les paramètres des résultats d'amarrage extraits à l'étape ① sont comme suit :

BE est énergie de liaison, égale à la somme de l'énergie intermoléculaire et de l'énergie libre de torsion ;
KI est la constante d'inhibition ;
ImE est l'énergie intermoléculaire, faisant référence à l'énergie entre les molécules ;
IE est énergie interne, faisant référence à l'énergie à l'intérieur des molécules ;
TE est l'énergie de torsion ;
UEE est l'énergie du système non lié ;
RR est le RMSD de référence, faisant référence à l'écart quadratique moyen de la structure actuelle par rapport à la conformation de référence ; où la probabilité gustative d'amertume, de douceur et d'aigreur de chaque composant monomère à l'étape ② est calculée en utilisant la formule suivante :

$$\hat{P}(k|x) = \frac{P(x|k)P(k)}{P(x)}$$

Parmi celles-ci, P (x|k) est l'équation de densité normale multivariée, dont la formule est comme suit :

$$P(x|k) = \frac{1}{\left((2\pi)^m |\Sigma_k|\right)^{1/2}} exp\left(-\frac{1}{2}(x - \mu_k)\Sigma_k^{-1}(x - \mu_k)^T\right)$$

x est la valeur du paramètre du résultat de l'ancrage d'un certain composant monomère, k est le groupe de goûts, y compris l'amertume, la douceur et l'aigreur ; m est le nombre de paramètres de résultat d'amarrage, où m=7 ;

$\Sigma_k$ est la covariance du k-ième groupe, $\mu_k$ est la moyenne du k-ième groupe, $|\Sigma_k|$ est la valeur de déterminant de $\Sigma_k$, $\Sigma_k^{-1}$ est la matrice inverse de $\Sigma_k$; $(x - \mu_k)^\tau$ est la matrice transposée ; les valeurs de covariance $\Sigma$ et de moyenne $\mu$ des différents groupes d'amertume, de douceur et d' aigreur sont comme suit :

| Groupe k | Paramètre | | Valeur | | | | | | |
|---|---|---|---|---|---|---|---|---|---|
| | | | BE | KI | ImE | IE | TE | UEE | RR |
| Amertume | $\Sigma$ | BE | 1.74 | 64.17 | 1.04 | -0.64 | 0.7 | -0.64 | 4.14 |
| | | KI | 64.17 | 48065.02 | 133.22 | 4.53 | -69.17 | 4.53 | 806.36 |
| | | ImE | 1.04 | 133.22 | 2.41 | 0.64 | -1.37 | 0.64 | 7.06 |
| | | IE | -0.64 | 4.53 | 0.64 | 0.97 | -1.28 | 0.97 | 1.11 |
| | | TE | 0.7 | -69.17 | -1.37 | -1.28 | 2.08 | -1.28 | -2.92 |
| | | UEE | -0.64 | 4.53 | 0.64 | 0.97 | -1.28 | 0.97 | 1.11 |
| | | RR | 4.14 | 806.36 | 7.06 | 1.11 | -2.92 | 1.11 | 48.32 |
| | $\mu$ | | -6.4 | 113.14 | -7.93 | -1.07 | 1.54 | -1.07 | 91.47 |
| Douceur | $\Sigma$ | BE | 1.36 | -105.06 | 1.06 | -1.06 | 0.3 | -1.06 | -1.23 |
| | | KI | -105.06 | 35402.7 | -173.76 | 63.16 | 68.57 | 63.16 | 76.49 |
| | | ImE | 1.06 | -173.76 | 3.29 | -0.04 | -2.22 | -0.04 | 1.35 |
| | | IE | -1.06 | 63.16 | -0.04 | 1.66 | -1.01 | 1.66 | -1.06 |
| | | TE | 0.3 | 68.57 | -2.22 | -1.01 | 2.52 | -1.01 | -2.57 |
| | | UEE | -1.06 | 63.16 | -0.04 | 1.66 | -1.01 | 1.66 | -1.06 |
| | | RR | -1.23 | 76.49 | 1.35 | -1.06 | -2.57 | -1.06 | 231.07 |
| | $\mu$ | | -3.04 | 119.05 | -6.43 | -3.42 | 3.39 | -3.42 | 89.44 |
| Aigreur | $\Sigma$ | BE | 0.23 | -39.3 | 0.86 | 0.04 | -0.63 | 0.04 | 1.14 |
| | | KI | -39.3 | 36008.6 | -169.87 | -9.21 | 130.57 | -9.21 | -210.71 |
| | | ImE | 0.86 | -169.87 | 4.19 | 0.2 | -3.33 | 0.2 | 5.82 |
| | | IE | 0.04 | -9.21 | 0.2 | 0.01 | -0.16 | 0.01 | 0.31 |
| | | TE | -0.63 | 130.57 | -3.33 | -0.16 | 2.7 | -0.16 | -4.68 |
| | | UEE | 0.04 | -9.21 | 0.2 | 0.01 | -0.16 | 0.01 | 0.31 |
| | | RR | 1.14 | -210.71 | 5.82 | 0.31 | -4.68 | 0.31 | 15.42 |
| | $\mu$ | | -3.23 | 47.4 | -5.2 | -0.11 | 1.97 | -0.11 | 93.55 |

$P(k)$ est la probabilité a priori du goût k avec les valeurs suivantes : $P(k$ = amertume) = 38,46%, $P(k$ = douceur) = 32,31%, $P(k$ = aigreur) = 29,23%,
$P(x)$ est une constante anormale, dont la formule est comme suit : $P(x) = \Sigma P(x \mid k) P(k)$.

**2.** La méthode selon la revendication 1, dans laquelle le calcul de la valeur gustative d'amertume, de douceur et d'aigreur de chaque composant monomère à l'étape ③ est comme suit :
La valeur gustative d'un certain monomère dans le mélange de composants = le contenu d'un monomère dans le mélange de composants × la probabilité gustative d'un monomère dans le mélange de composants.

**3.** La méthode selon la revendication 1, dans laquelle le calcul des caractéristiques gustatives du mélange de composants à l'étape @ est comme suit :
La probabilité gustative globale d'amertume, de douceur et d'aigreur du mélange de composants = somme des valeurs gustatives du mélange de composants/somme du contenu du mélange de composants.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112102886 A

**Non-patent literature cited in the description**

- **HU KE et al.** Exploring the Mechanism of Liquid Smoke and Human Taste Perception Based on the Synergy of the Electronic Tongue, Molecular Docking, and Multiple Linear Regression. *FOOD BIO-PHYSICS*

- **GOEL ANUKRA.TI et al.** In-silico screening of database for finding potential sweet molecules: A combined data and structure based modeling approach. *FOOD CHEMISTRY*
- **RUDRAKSH TUWANI et al.** BitterSweet: Building machine learning models for predicting the bitter and sweet taste of small molecules. *SCIENTIFIC REPORTS*